# EUROPEAN PATENT SPECIFICATION

(11) **EP 1 847 238 B1**
(45) Date of publication and mention of the grant of the patent: **09.09.2009**
(21) Application number: 06121315.3
(22) Date of filing: 27.09.2006
(51) Int. Cl.: A61F 2/36

(54) **Femoral prosthesis**
Femurprothese
Prothèse fémorale

(30) Priority: 18.04.2006 IT VI20060115
(43) Date of publication of application: 24.10.2007
(73) Proprietor: Plus Orthopedics Italy Srl, 20041 Agrate Brianza (MB) (IT)
(72) Inventor: CURRADINI, Giorgio, 20123, MILANO (IT); LANFRANCO, Stefano, 20153, MILANO (IT)
(74) Representative: Bonini, Ercole

(56) References cited:
- EP-A- 0 669 116
- EP-A1- 0 682 924
- EP-A1- 0 978 262
- EP-A2- 0 677 281
- DE-A1- 2 607 315

## Description

The present invention relates to a prosthesis particularly suitable for implantation in the femur.

It is common knowledge that degeneration of the human lower limbs can affect the hip joint at the point where the head of the femur forms an articulation with the acetabulum in the iliac bone.

Such degeneration can cause pain and restrict motion, making the person inclined to limp and have difficulty moving the lower limb.

In the more complex cases, such as in the presence of dysplasia or arthritis, the solution to these problems lies in replacing the bone joint with an artificial joint consisting of a prosthesis implanted in lieu of the head of the femur and forming a mobile articulation with an acetabular unit attached to the acetabulum.

The femoral prostheses of known type typically have a shaped stem that fits into a corresponding canal created in the femur.

Clearly, the stability of the coupling between the bone and the prosthesis is of fundamental importance because any mobility of the latter would be a constant cause of pain to the patient, making it necessary to replace the prosthesis.

To afford the necessary stability, two types of prosthesis are used, i.e. cemented and uncemented.

The former are attached to the femur with the aid of a filler, adhesive or cement, while the latter are shaped so as to facilitate bone regrowth and its consequent attachment.

In this latter case, given the morphology of human bone, the prosthesis is made with a surface shape designed to guarantee an initial stability of the prosthesis inside the femur when it is loaded (primary stability), giving the bone time to grow back and become permanently attached to the prosthesis (secondary stability).

For this purpose, the prostheses of known type have differently-shaped cross-sections, among them (especially for femoral implants) are the prostheses with a circular cross-section.

By comparison with other shapes, this has the advantage of enabling the orientation of the prosthesis inside the femoral canal to be adjusted even after said canal has been prepared, thus ensuring a greater intra operative flexibility than prostheses with an asymmetrical cross-section.

Prostheses with a circular cross-section must be provided, however, with a plurality of torsion-resistant fins projecting from the lateral surface of the stem that generate a plurality of corresponding grooves in the bone, which thus oppose the rotational movement to which the prosthesis is submitted when the joint in use.

*An example of said fins* is disclosed in the European Patent application no. EP-A-0 677 281 which discloses the features of the preamble of claim 1.

Although their application is quite straightforward, these bone prostheses nonetheless have several acknowledged drawbacks.

The first of these drawbacks lies in that, despite the presence of the torsion-resistant fins, the regrowing bone does not always bond adequately with the prosthesis, in which case the latter has to be replaced.

In some cases, moreover, the torsion-resistant fins are not sufficient to contrast the rotational stress at the femur-prosthesis interface, allowing the prosthesis to move inside the bone and consequently preventing the bone's regrowth.

The object of the present invention is to overcome the aforementioned drawbacks.

In particular, a first object of the present invention is to produce a femoral prosthesis with a substantially circular, symmetrical cross-section that facilitates the bonding of the regrowing bone to the prosthesis better than equivalent prostheses of the known state of the art.

Another object of the present invention is to produce a femoral prosthesis that affords a greater resistance to torsional stress than equivalent prostheses belonging to the known state of the art.

The aforementioned objects are achieved by a bone prosthesis as described in the main claim; the dependent claims contain details of the features of the invention.

The presence of ridges between each adjacent pair of torsion-resistant fins advantageously increases the bone's capacity for gripping and bonding to the prosthesis.

It is common knowledge, in fact, that the bone is more stimulated to bond to an object if said object has a marked surface roughness.

A further advantage lies in that the number of said ridges can be increased by increasing the number of straight portions coming between each pair of fins.

When the prosthesis is implanted in the femur, said ridges advantageously generate seats and thereby further contrast the torsional stress coming to bear on the prosthesis-femur interface.

Moreover, being recessed towards the inside of the shaped stem, the connecting portions between the straight portions and the adjacent fins advantageously increase the surface area of contact between the bone and the prosthesis by comparison with equivalent prostheses of the known state of the art.

Increasing the contact area at the interface increases both the capacity of the prosthesis to withstand torsional stresses and the capacity of the bone to bond to the prosthesis.

The aforesaid objects and advantages, as well as others that will be better illustrated in the following pages, are explained in more detail in the description of a preferred embodiment, provided by way of a non-restrictive example with reference to the attached drawings wherein:
- fig. 1 shows the femoral prosthesis of the invention in an axonometric view;
- fig. 2 shows an exploded view of the femoral prosthesis of the invention in an example of its application;
- fig. 3 shows a cross-section of the prosthesis of fig. 1;
- figs. 4 and 5 show cross-sections of several embodiments of the prosthesis of the invention.

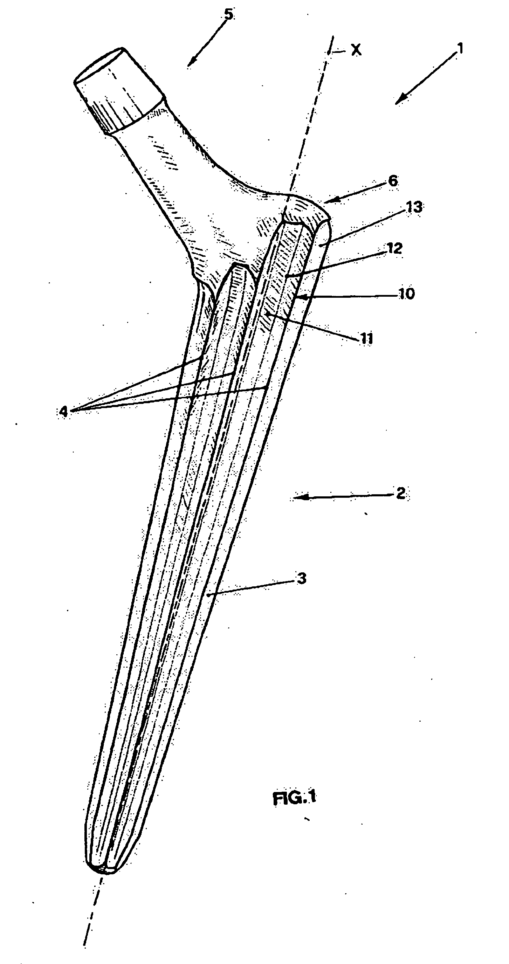

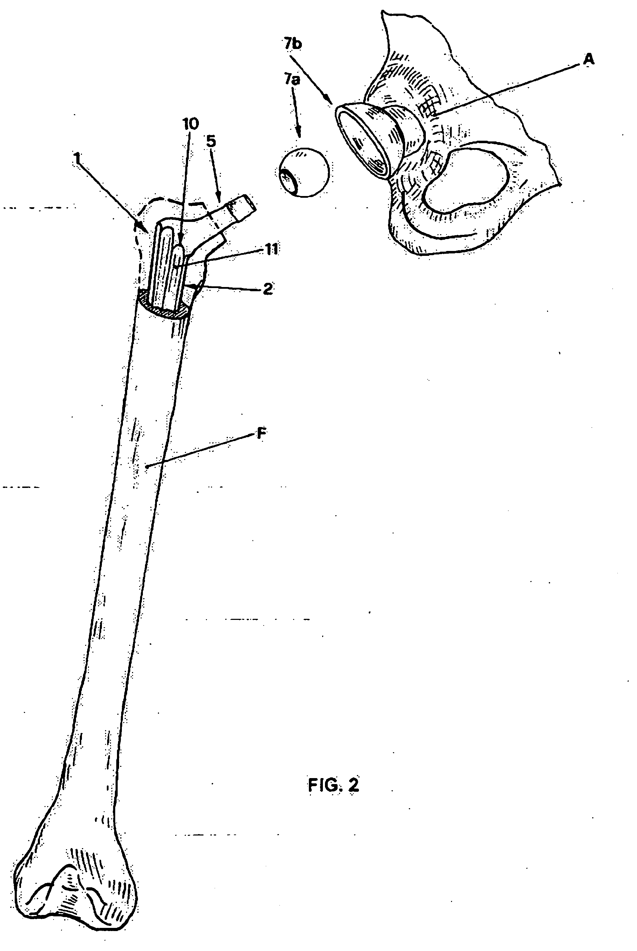

The femoral prosthesis forming the object of the invention is illustrated in figures 1 and 2, where it is globally indicated by the numeral **1** and where it can be seen to comprise at least one stem **2** with a substantially truncated cone shape, the lateral surface **3** of which is shaped into a plurality of torsion-resistant fins **4** running in the longitudinal direction **X** along said shaped stem **2.**

The femoral prosthesis **1** also comprises an appendage **5** projecting from the top base **6** of the shaped stem **2** designed to support a spherical element **7a** that forms an articulation with an acetabular element **7b** associated with the acetabulum **A** of the user.

According to the invention, the peripheral portion **10** of any cross-section of the shaped stem **2,** coming between any pair of adjacent fins **4,** comprises at least one substantially straight portion **11** connected to at least one of the fins **4** by means of a recessed connecting portion **13,** i.e. that forms a groove in the shaped stem **2** and that, together with said straight portion **11,** forms a ridge **12.**

This configuration advantageously gives rise to a plurality of ridges generated along the longitudinal axis **X** of the shaped stem **2,** which stimulate the bone ingrowth of the femoral bone **F** around prosthesis **1.**

Moreover, the recessed configuration of said connecting portion **13** advantageously enables an increase in the contact surface area at the interface between the bone **F** and the prosthesis **1,** thereby improving the stability of the latter by comparison with equivalent prostheses of known type.

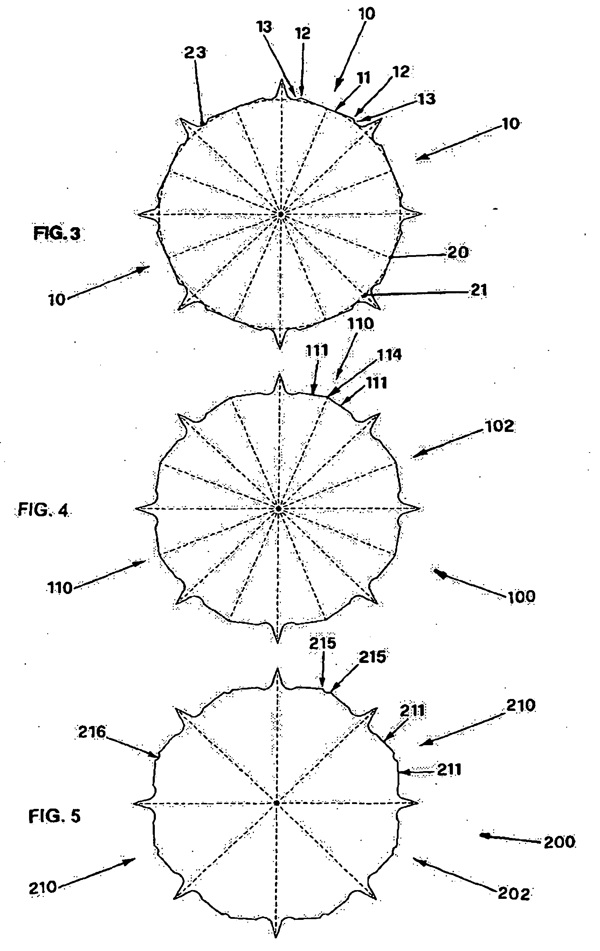

According to the preferred embodiment described herein, there is a single substantially straight portion **11,** that spatially forms a substantially trapezoid-shaped surface with its top base facing towards the appendage **5** of the prosthesis **1** and its bottom base facing towards the inside of the femur **F** in which the prosthesis is inserted, for each peripheral portion coming between two adjacent fins **4,** as shown in the cross-section in fig. 3.

Said embodiment is not intended to restrict the possibility of alternative embodiments, however, wherein the peripheral portion **110** comprises two or more substantially straight portions **111,** which, between them, form at least one additional ridge **114,** as illustrated in fig. 4.

According to another, different embodiment illustrated in the example in fig. 5, there may be several ridges **215** between the straight portions **211** coming between two adjacent fins **204.**

Moreover, said ridges may be connected together by curved portions **216** that are preferably recessed towards the inside of the shaped stem **202,** here again to enable an increase in the surface area of contact between the bone **F** and the prosthesis **200.**

Returning to the preferred embodiment, all the straight portions **11** clearly have a point of tangency **20** on the same circumference **21** and, to be more precise, said point of tangency **20** is the midpoint along the corresponding straight portion **11.**

As for the connecting portion **13,** this preferably comprises at least one curved part **23** that, as previously mentioned, enables an increase in the contact surface area between the bone **F** and the prosthesis **1.**

To be more precise, the curved part **23** is convex, or recessed, towards the inside of the shaped stem **2,** although this is not intended to restrict any other embodiments wherein the second connecting portion may not necessarily include a curved part, but form a mixtilinear connection that is nonetheless recessed towards the inside of said shaped stem.

Finally, the fins **4** preferably, but not necessarily, have a cross-section in the shape of a triangle.

To be more specific, the sloping sides of the triangle may be straight or curved, with their convexity facing towards the inside of the triangle to accentuate their torsion-resistant characteristic.

The shape of the fins' cross-section may differ in other embodiments (not illustrated herein) and may, for instance, be quadrilateral.

In practical terms, during the surgical procedure, the surgeon cuts the head of the femur and prepares a circular seat therein.

Then the femoral prosthesis **1** of the invention is inserted in said seat, oriented at a suitable angle.

As the stem is inserted, the fins **4** and ridges **12** dig into the sides of said seat, creating corresponding grooves in which they exert their torsion-resistant effect.

Finally, the appendage **5** is inserted in the ball-shaped element **7a,** which forms an articulated joint in association with the acetabular unit **7b** in the acetabulum.

In the postoperative period, the fins **4** and ridges **12** ensure that the prosthesis **1** remains sufficiently stable, enabling the regrowth of the bone **F,** which becomes coupled to the prosthesis **1.**

In the light of the above considerations, the femoral prosthesis of the invention achieves all the previously-stated objects.

In particular, the femoral prosthesis of the invention achieves the object of facilitating the adhesion of the bone to the prosthesis by comparison with equivalent prostheses belonging to the known state of the art thanks to the presence on its lateral surface of a plurality of ridges that, as explained earlier, accentuate the capacity for the bone to adhere to the prosthesis.

The object of producing a femoral prosthesis that affords a greater resistance to torsional stress than prostheses of the known state of the art is also achieved.

In the executive stages, the femoral prosthesis of the invention may undergo further additions or variations that will also be covered by the present patent if they come within the scope of the claims that follow, even if they are not illustrated or described herein.

Where technical features mentioned in any claim are followed by reference signs, those reference sings have been included for the sole purpose of increasing the intelligibility of the claims and accordingly such reference signs do not have any limiting effect on the interpretation of each element identified by way of example by such reference signs.

## Claims

1. Femoral prosthesis (1; 100; 200) *comprising:*
- a shaped stem (2,102; 202) with a substantially truncated one shape,
- a plurality of torsion-resistant fins (4) projecting from the lateral surface (3) of said shaped stem (2; 102; 202) and extending in the longitudinal direction (X) along said shaped stem (2; 102; 202),
*wherein* the peripheral portion (10; 110; 210) of any cross-section of said shaped stem (2; 102; 202) coming between any adjacent pair of said fins (4) comprises at least one substantially straight portion (11; 111; 211) ***characterized in that** said straight portion (11;111;211) is* connected to at least one of said fins (4) by means of a connecting portion (13) that is recessed towards the inside of said shaped stem (2; 102; 202), said connecting portion (13) thereby defining a ridge (12) in relation to said straight portion (11; 111; 211).

2. Femoral prosthesis (100; 200) according to claim 1), **characterized in that** said peripheral portion (110; 210) comprises at least two substantially straight portions (111; 211) forming at least one ridge (114; 215) between them.

3. Femoral prosthesis (200) according to claim 2), **characterized in that** there are two or more additional ridges (215) coming between said at least two substantially straight portions (211), which are connected together by curved portions (216) recessed towards the inside of said shaped stem (202).

4. Femoral prosthesis (1; 100; 200) according to claim 1), **characterized in that** all of said straight portions (11; 111; 211) have a point of tangency (20) on the same circumference (21).

5. Femoral prosthesis (1; 100; 200) according to claim 4), **characterized in that** said point of tangency (20) is the midpoint along the corresponding straight portion (11; 111; 211).

6. Femoral prosthesis (1; 100; 200) according to claim 1), **characterized in that** said connecting portion (13) comprises at least one curved part (23).

7. Femoral prosthesis (1; 100; 200) according to claim 3) or 6), **characterized in that** said curved part (23; 216) has convex surfaces recessed towards the inside of said shaped stem (2; 102; 202).

8. Femoral prosthesis (1; 100; 200) according to claim 1), **characterized in that** said fins (4) have a cross-section of substantially triangular shape.

9. Femoral prosthesis (1; 100; 200) according to claim 1), **characterized in that** the sloping sides of said triangle are curved.

## Patentansprüche

1. Oberschenkelprothese (1; 100; 200), umfassend:
- einen geformten Schaft (2; 102; 202) mit einer im Wesentlichen kegelstumpfförmigen Form; und
- eine Vielzahl an torsionsbeständigen Rippen (4), die von der Seitenoberfläche (3) des geformten Schafts (2; 102; 202) vorstehen und sich in Längsrichtung (X) entlang dem geformten Schaft (2; 102; 202) erstrecken;
wobei der Umfangsrandabschnitt (10; 110; 210) eines jeden Querschnitts des geformten Schafts (2; 102; 202), der zwischen jedem benachbarten Paar aus Rippen (4) vorhanden ist, zumindest einen im Wesentlichen geraden Abschnitt (11; 111; 211) umfasst, **dadurch gekennzeichnet, dass** der gerade Abschnitt (11; 111; 211) über einen Verbindungsabschnitt (13), der zum Inneren des geformten Schafts (2; 102; 202) hin vertieft ist, mit zumindest einer der Rippen (4) verbunden ist, wobei der Verbindungsabschnitt (13) dadurch in Bezug auf den geraden Abschnitt (11; 111; 211) eine Kante (12) definiert.

2. Oberschenkelprothese (100; 200) nach Anspruch 1), **dadurch gekennzeichnet, dass** der Umfangsrandabschnitt (110; 210) zumindest zwei im Wesentlichen gerade Abschnitte (111; 211) umfasst, die zumindest eine Kante (114; 215) zwischen sich definieren.

3. Oberschenkelprothese (200) nach Anspruch 2), **dadurch gekennzeichnet, dass** zwei oder mehr zusätzliche Kanten (215) zwischen den zumindest zwei im Wesentlichen geraden Abschnitten (211) vorhanden sind, die durch gebogene Abschnitte (216), die zum Inneren des geformten Schafts (202) hin vertieft sind, miteinander verbunden sind.

4. Oberschenkelprothese (1; 100; 200) nach Anspruch 1), **dadurch gekennzeichnet, dass** alle geraden Abschnitte (11; 111; 211) einen Tangentenpunkt (20) am gleichen Umfang (21) aufweisen.

5. Oberschenkelprothese (1; 100; 200) nach Anspruch 4), **dadurch gekennzeichnet, dass** der Tangentenpunkt (20) der Mittelpunkt entlang dem entsprechenden geraden Abschnitt (11; 111; 211) ist.

6. Oberschenkelprothese (1; 100; 200) nach Anspruch 1), **dadurch gekennzeichnet, dass** der Verbindungsabschnitt (13) zumindest einen gebogenen Teil (23) umfasst.

7. Oberschenkelprothese (1; 100; 200) nach Anspruch 3) oder 6),
**dadurch gekennzeichnet, dass** der gebogene Teil (23; 216) konvexe Oberflächen aufweist, die zum Inneren des geformten Schafts (2; 102; 202) hin vertieft sind.

8. Oberschenkelprothese (1; 100; 200) nach Anspruch 1), **dadurch gekennzeichnet, dass** die Rippen (4) einen Querschnitt von im Wesentlichen dreieckiger Form aufweisen.

9. Oberschenkelprothese (1; 100; 200) nach Anspruch 1), **dadurch gekennzeichnet, dass** die schrägen Seiten des Dreiecks gebogen sind.

## Revendications

1. Prothèse fémorale (1; 100; 200) comprenant:
- une tige galbée (2; 102; 202) ayant une forme essentiellement tronconique;
- une pluralité d'ailettes anti-torsion (4) saillant de la surface latérale (3) de ladite tige galbée (2; 102; 202) et s'étendant dans la direction longitudinale (X) le long de ladite tige galbée (2; 102 ; 202);
où la partie périphérique (10; 110; 210) de n'importe quelle section transversale de ladite tige galbée (2; 102; 202), positionnée de manière adjacente entre un couple quelconque desdites ailettes (4) comprend au moins une partie essentiellement droite (11; 111; 211) **caractérisée en ce que** ladite partie droite (11; 111; 211) est reliée à au moins une desdites ailettes (4) au moyen d'une partie de connexion (13) qui est reculée vers l'intérieur de ladite tige galbée (2; 102; 202), ladite partie de connexion (13) définissant une arête (12) avec ladite partie droite (11; 111; 211).

2. Prothèse fémorale (100; 200) selon la revendication 1), **caractérisée en ce que** ladite partie périphérique (110; 210) comprend au moins deux parties essentiellement droites (111; 211) formant au moins une arête (114; 215) entre celles-ci.

3. Prothèse fémorale (200) selon la revendication 2), **caractérisée en ce qu'**il y a deux ou plusieurs arêtes ultérieures (215) se positionnant entre lesdites au moins deux arêtes essentiellement droites (211), qui sont reliées ensemble par des parties courbes (216) reculées vers l'intérieur de ladite tige galbée (202).

4. Prothèse fémorale (1; 100; 200) selon la revendication 1), **caractérisée en ce que** toutes ledites parties droites (11; 111; 211) présentent un point tangent (20) sur la même circonférence (21).

5. Prothèse fémorale (1; 100; 200) selon la revendication 4), **caractérisée en ce que** ledit point tangent (20) est le point médian le long de la partie droite correspondante (11; 111; 211).

6. Prothèse fémorale (1; 100; 200) selon la revendication 1), **caractérisée en ce que** ladite partie de connexion (13) comprend au moins une partie courbe (23).

7. Prothèse fémorale (1; 100; 200) selon la revendication 3) ou 6), **caractérisée en ce que** ladite partie courbe (23; 216) présente des surfaces convexes reculées vers l'intérieur de ladite tige galbée (2; 102; 202).

8. Prothèse fémorale (1; 100; 200) selon la revendication 1), **caractérisée en ce que** ledites ailettes (4) présentent une section transversale de forme essentiellement tringulaire.

9. Prothèse fémorale (1; 100; 200) selon la revendication 1), **caractérisée en ce que** les côtés inclinés dudit triangle sont courbes.
